# FASCICULE DE BREVET EUROPEEN

(11) **EP 1 192 932 B1**
(45) Date de publication et mention de la délivrance du brevet: **21.03.2007**
(21) Numéro de dépôt: 01402446.7
(22) Date de dépôt: 24.09.2001
(51) Int. Cl.: A61K 8/90, A61Q 5/06

(54) **Gel coiffant longue tenue**
Dauerhaftes Haarfrisurgel
Long-wear hair-styling gel

(30) Priorité: 02.10.2000 FR 0012515
(43) Date de publication de la demande: 03.04.2002
(73) Titulaire: L'ORÉAL, 75008 Paris (FR)
(72) Inventeur: Belli, Emmanuelle, 92600 Asnières (FR)
(74) Mandataire: Casalonga, Axel

(56) Documents cités:
- WO-A-00/40628
- US-A- 5 986 015

## Description

La présente invention concerne une composition cosmétique épaissie par une combinaison d'au moins deux polymères épaississants, ainsi que l'utilisation de cette composition pour le coiffage des cheveux.

Les gels coiffants sont des solutions d'un ou de plusieurs polymères filmogènes fixants, épaissies ou gélifiées par un ou plusieurs polymères épaississants.
Bien que l'on connaisse dans le domaine du coiffage un très grand nombre de polymères fixants, la plupart d'entre eux présentent un pouvoir fixant limité dans le temps et qui résiste mal à l'humidité.

L'utilisation dans le domaine cosmétique de polymères greffés est par ailleurs connue, comme en atteste le brevet US 5 986 015, qui décrit un nouveau procédé de préparation, en deux étapes, de polymères greffés hydrophiles ou hydrophobes susceptibles notamment d'être employés dans des produits de fixation capillaire.

On a découvert récemment les propriétés de coiffage très intéressantes d'un groupe particulier de copolymères à blocs ramifiés.
Ces copolymères, utilisés dans des compositions de coiffage, présentent une combinaison de propriétés physicochimiques et cosmétiques qui en font d'excellents polymères fixants. Ainsi, ces copolymères séquencés s'étalent facilement sur les cheveux, présentent une bonne adhésion aux fibres capillaires, donnent un toucher peu collant, s'éliminent facilement au shampooing et donnent une fixation satisfaisante d'une bonne élasticité stable dans le temps et qui résiste particulièrement bien à l'humidité.

Or, on a constaté lors de la formulation de gels coiffants, que ces nouveaux polymères avaient pour inconvénient de fluidifier considérablement la plupart des systèmes épaississants classiques comme par exemple ceux à base d'homopolymères ou de copolymères acryliques.
Une augmentation de la viscosité de la composition par accroissement de la concentration du polymère épaississant s'est avérée insatisfaisante en raison des mauvaises qualités d'usage du gel qui en résultaient, telles qu'une consistance pâteuse et collante et une certaine difficulté d'étalement et d'application du gel.

Il existait donc un besoin d'un système épaississant capable d'épaissir ou de gélifier de manière satisfaisante une composition coiffante à base des nouveaux copolymères fixants indiqués ci-dessus.

La demanderesse a trouvé, après de longues recherches, une association particulière de polymères épaississants permettant de surmonter les problèmes de fluidification et d'altération des propriétés d'usage du gel exposés ci-dessus

La présente invention a par conséquent pour objet des compositions cosmétiques contenant dans un support cosmétiquement acceptable
(a) au moins un polymère filmogène fixant choisi parmi les copolymères à blocs ramifiés comprenant, comme monomères principaux, au moins un acrylate d'alkyle en C₁₋₂₀ et/ou au moins un N-mono- ou N,N-di-(alkyle en C₂₋₁₂)(méth)acrylamide, et de l'acide acrylique et/ou de l'acide méthacrylique.
(b) au moins un agent épaississant choisi parmi les homopolymères et copolymères à base d'acide (méth)acrylique, réticulés ou non réticulés, et
(c) au moins un co-épaississant choisi parmi les polymères épaississants non cellulosiques différents de (b).

L'invention a également pour objet l'utilisation des compositions cosmétiques épaissies ou gélifiées ci-dessus pour le coiffage et la fixation des cheveux.

Le polymère filmogène fixant (a) utilisé dans les compositions cosmétiques de la présente invention est un copolymère à blocs ramifié ayant une structure constituée de blocs hydrophobes sur lesquels sont fixées, notamment par l'intermédiaire de motifs bifonctionnels, un certain nombre de blocs plus hydrophiles. Ces copolymères présentent au moins deux températures de transition vitreuse.

Des exemples de polymères filmogènes fixants (a) susceptibles d'être employés dans les compositions cosmétiques selon la présente invention, ainsi que leurs procédés de préparation, sont entre autres décrits dans la demande de brevet WO 00/40628.

Les copolymères séquencés ramifiés décrits ci-dessus sont proposés par exemple sous les dénominations EX-SDR-26^{®} et EX-SDR-45^{®} par la société GOODRICH.

Ces copolymères présentent la composition suivante :
Acide acrylique 26 à 36 % en moles
Acrylate de n-butyle 27,5 à 30,5 % en moles
Acide méthacrylique 33,3 à 45,3 % en moles
Méthacrylate d'allyle 0,48 à 0,92 % en moles

Les blocs les plus hydrophobes ont un poids moléculaire de 10 000 à 100 000 et les blocs les plus hydrophobes ont un poids moléculaire de 1000 à 100 000 daltons.

Les polymères filmogènes fixants ci-dessus sont utilisés de préférence sous forme anionique, c'est-à-dire sous forme de sel résultant de la neutralisation partielle ou totale des groupes acide (méth)acrylique. L'agent de neutralisation peut être n'importe qu'elle base minérale ou organique physiologiquement acceptable qui n'interfère pas de manière désavantageuse avec le système épaississant. On peut citer à titre d'exemple d'agent de neutralisation préféré le 2-amino-2-méthyl-1-propanol ou l'hydroxyde de sodium.

Les compositions cosmétiques de la présente invention contiennent généralement entre 0,1 et 10 % en poids, et de préférence entre 1 et 5 % en poids de polymère filmogène fixant, rapporté au poids total de la composition finale.

Le système épaississant utilisé dans les compositions cosmétiques de la présente invention comprend nécessairement
(b) au moins un polymère épaississant à base d'acide (méth)acrylique, et
(c) au moins un polymère épaississant non cellulosique (co-epaississant), différent de (b).

Le polymère épaississant (b) est choisi parmi le poly(acide acrylique), le poly(acide méthacrylique), les copolymères d'acide acrylique et d'acide méthacrylique, les copolymères comprenant des motifs dérivés d'acide acrylique et/ou d'acide méthacrylique ainsi que des motifs dérivés d'autres monomères acryliques ou vinyliques tels que les acrylates d'alkyle en C₁₋₃₀, les méthacrylates d'alkyle en C₁₋₃₀, l'acétate de vinyle.
Ces homopolymères ou copolymères acryliques peuvent également être réticulés.

Ils sont utilisés sous forme partiellement ou totalement neutralisée par une base organique ou minérale physiologiquement acceptable.

Dans un mode de réalisation préféré de l'invention, le polymère (b) est un homopolymère réticulé d'acide méthacrylique ou d'acide acrylique.
On peut citer à titre d'exemples de tels polymères ceux commercialisés par la société GOODRICH sous les dénominations Carbopol^{®} 940, Carbopol^{®} 941, Carbopol^{®} 980, Carbopol^{®} 981, Carbopol^{®} ETD 2001, Carbopol^{®} ÉTD 2050, Carbopol^{®} 2984, Carbopol^{®} 5984 et Carbopol^{®} Ultrez 10, par la société 3V sous les dénominations Synthalen^{®} K, Synthalen^{®} L et Synthalen^{®} MS, et par la société PROTEX sous les dénominations Modarez^{®} V1250 PX, Modarez^{®} V2000 PX, Viscaron^{®} A1600 PE et Viscaron^{®} A700 PE.
Dans un autre mode de réalisation préféré de l'invention, l'agent épaississant (b) est un copolymère d'acide acrylique ou d'acide méthacrylique et d'un acrylate ou méthacrylate d'alkyle en C₁₀₋₃₀.
De tels copolymères sont commercialisés par exemple par la société GOODRICH sous les dénominations Carbopol^{®} 1342, Carbopol^{®} 1382, Pemulen^{®} TR1 ou et Pemulen^{®} TR2.

La concentration en agent épaississant (b) des compositions cosmétiques de la présente invention est de préférence comprise entre 0,1 et 3 % en poids, et en particulier entre 0,2 et 2 % en poids, rapporté au poids total de la composition finale.

Pour l'obtention d'un effet épaississant satisfaisant, c'est-à-dire pour l'obtention d'une viscosité au moins égale à 50 unités de déviation au viscosimètre RHEOMAT 180, mobile 3 (25 °C, lecture après 30 secondes), soit environ 1,9 Pa.s, ce premier constituant épaississant (b) doit être associé à un deuxième constituant (c) jouant le rôle de co-épaississant.

Ce co-épaississant est choisi parmi les polymères épaississants différents des épaississants (b), à l'exclusion des polymères épaississants cellulosiques.

Selon un mode de réalisation préféré de la présente invention, ce co-épaississant (c) est un polymère d'origine naturel non cellulosique.

On peut citer à titre de polymères épaississants d'origine naturelle, utilisables en tant que co-épaississants, les gommes de guar, de xanthane, de scléroglucane, de gellane, de rhamsane et de karaya, les alginates, la maltodextrine, l'amidon et ses dérivés, et la farine de caroube, et on préfère utiliser en particulier les gommes de guar telles que celle commercialisée sous la dénomination Jaguar^{®} HP105 par la société RHODIA, ou les gommes de xanthane telles que celles commercialisées sous les dénominations Keltrol^{®} et Kelza^{®} par la société MONSANTO, ou sous la dénomination Rhodopol^{®} par la société RHODIA.

Comme co-épaississants synthétiques, on peut utiliser les polyéthylèneglycols et leurs dérivés, ainsi que les homopolymères et copolymères, réticulés ou non, à base d'acrylamide ou de méthacrylamide tels que les homopolymères d'acide 2-acrylamido-2-méthylpropanesulfonique, les copolymères d'acrylamide ou de méthacrylamide et de chlorure de méthacryloyloxyéthyltriméthylammonium, ou les copolymères d'acrylamide et d'acide 2-acrylamido-2-méthylpropanesulfonique.

La concentration du co-épaississant dans les compositions cosmétiques selon la présente invention est de préférence comprise entre 0,05 et 2 % en poids, et en particulier entre 0,1 et 1 % en poids, rapporté au poids total de la composition finale.

On utilise comme support cosmétiquement acceptable dans les compositions cosmétiques de la présente invention un support capable de solubiliser les constituants polymériques (a), (b) et (c) décrits ci-dessus, et il s'agit d'un support aqueux ou hydroalcoolique.

Les compositions cosmétiques de la présente invention peuvent contenir en outre d'autres ingrédients utilisés couramment dans le domaine cosmétique et appropriés à l'application envisagée. On peut citer à titre d'exemple de tels additifs par exemple des colorants, des pigments, des parfums, des silicones volatiles ou non, organomodifiées ou non, des filtres solaires, des polymères fixants anioniques, non ioniques, cationiques ou amphotères, différents de ceux décrits ci-dessus (tels que par exemple la polyvinylpyrrolidone), pour autant qu'ils n'altèrent pas les propriétés avantageuses des compositions cosmétiques de la présente invention.

La présente invention est illustrée à l'aide de l'exemple suivant.

### Exemple

On prépare les gels coiffants hydro-alcooliques A, B et C contenant les ingrédients suivants (en parties en poids) :

| | **A** | **B** | **C** |
|---|---|---|---|
| polymère fixant^{a)} | 0,2 | 0,2 | 0,2 |
| polymère épaississant acrylique^{b)} | 1,4 | 1,4 | 1,4 |
| co-épaississant non cellulosique^{c)} | 0,3 | | |
| co-épaississant cellulosique^{d)} | | 0,3 | |
| silicone^{e)} | 0,2 | 0,2 | 0,2 |
| éthanol à 96° | 17,2 | 17,2 | 17,2 |
| 2-amino-2-méthylpropanol-1 | qsp pH 7,5 | qsp pH 7,5 | qsp pH 7,5 |
| eau | qsp 100 | qsp 100 | qsp 100 % |
| viscosité^{f)} | 2,540 | 1,480 | 1,535 |

| | | | |
|---|---|---|---|
| a) Ex-SDR-26^{®}, copolymère séquencé ramifié d'acrylate de butyle et d'acides (méth)acryliques commercialisé par la société GOODRICH b) Carbopol^{®} Ultrez 10, poly(acide acrylique) commercialisé par la société GOODRICH c) Jaguar^{®} HP 105, gomme de guar commercialisée par la société RHODIA d) Klucel EF^{®}, hydroxypropylcellulose (en moyenne 22 moles d'oxyde d'éthylène et 23 moles d'oxyde de propylène) commercialisée par la société AQUALON e) Mirasil^{®} DMCO, polydiméthylsiloxane commercialisé par la société RHODIA f) viscosité exprimée en Pa.s, mesurée à 25 °C à l'aide d'un viscosimètre rotatif RHEOMAT 180, mobile n° 3, lecture au bout de 30 secondes. | | | |

Cet exemple montre que le gel coiffant A selon la présente invention possède une viscosité sensiblement plus élevée que le gel coiffant comparatif C exempt d'agent co-épaississant et contenant, comme seul agent épaississant, un polymère épaississant acrylique (Carbopol^{®} Ultrez 10).

La comparaison du gel coiffant A selon la présente invention avec le gel coiffant comparatif B contenant un polymère épaississant acrylique (Carbopol^{®} Ultrez 10) associé à un co-épaississant cellulosique (Klucel^{®}), montre que le remplacement d'un co-épaississant cellulosique par un co-épaississant non cellulosique améliore de façon spectaculaire la viscosité du gel coiffant.

## Revendications

1. Composition cosmétique contenant dans un support cosmétiquement acceptable aqueux ou hydroalcoolique :
(a) au moins un polymère filmogène fixant choisi parmi les copolymères à blocs ramifiés ayant une structure constituée de blocs hydrophobes sur lesquels sont fixés par l'intermédiaire de motifs bifonctionnels un certain nombre de blocs hydrophiles, et qui comprennent comme monomères principaux, au moins un acrylate d'alkyle en C₁₋₂₀ et/ou au moins un N-mono- ou N,N-di-(alkyle en C₂₋₁₂)(méth)acrylamide, et de l'acide acrylique et/ou de l'acide méthacrylique,
(b) au moins un agent épaississant choisi parmi les homopolymères et copolymères à base d'acide (méth)acrylique, réticulés ou non réticulés, et
(c) au moins un co-épaississant différent de (b) choisi parmi les polymères épaississants ci-après :
- les polymères d'origine naturelle non cellulosiques,
- les co-épaississants synthétiques choisis parmi les polyéthylèneglycols et leurs dérivés ainsi que les homopolymères et copolymères, réticulés ou non, à base d'acrylamide ou de méthacrylamide.

2. Composition cosmétique selon la revendication 1, **caractérisée par le fait que** le polymère filmogène fixant est un copolymère à blocs ramifié comprenant, comme monomères, de l'acrylate de n-butyle, de l'acide acrylique, de l'acide méthacrylique et du méthacrylate d'allyle.

3. Composition cosmétique selon la revendication 1 ou 2, **caractérisée par le fait que** la concentration du polymère filmogène fixant est comprise entre 0,1 et 10 % en poids, de préférence entre 1 et 5 % en poids, rapporté au poids total de la composition finale.

4. Composition cosmétique selon l'une quelconque des revendications précédentes, **caractérisée par le fait que** l'agent épaississant (b) est un homopolymère d'acide acrylique réticulé.

5. Composition cosmétique selon l'une des revendications 1 à 3, **caractérisée par le fait que** l'agent épaississant (b) est un copolymère d'acide acrylique ou d'acide méthacrylique et d'un acrylate ou méthacrylate d'alkyle en C₁₀₋₃₀.

6. Composition cosmétique selon l'une quelconque des revendications précédentes, **caractérisée par le fait que** la concentration de l'agent épaississant (b) est comprise entre 0,1 et 3 % en poids, et de préférence entre 0,2 et 2 % en poids, rapporté au poids total de la composition finale.

7. Composition cosmétique selon l'une quelconque des revendications précédentes, **caractérisée par le fait que** le co-épaississant (c) est d'origine naturelle et est choisi parmi les gommes de guar, de xanthane, de scléroglucane, de gellane, de rhamsane et de karaya, les alginates, la maltodextrine, l'amidon et ses dérivés, et la farine de caroube.

8. Composition cosmétique selon la revendication 7, **caractérisée par le fait que** le co-épaississant (c) est choisi parmi la gomme de guar ou la gomme de xanthane.

9. Composition cosmétique selon l'une quelconque des revendications précédentes, **caractérisée par le fait que** la concentration du co-épaississant est comprise entre 0,05 et 2 % en poids, de préférence entre 0,1 et 1 % en poids, rapporté au poids total de la composition finale.

10. Utilisation des compositions cosmétiques selon l'une quelconque des revendications précédentes pour le coiffage et la fixation des cheveux.

## Claims

1. Cosmetic composition containing in an aqueous or aqueous-alcoholic cosmetically acceptable carrier
(a) at least one fixing film-forming polymer chosen from branched block copolymers having a structure consisting of hydrophobic blocks onto which a certain number of hydrophilic blocks are attached via bifunctional units and which comprise, as principal monomers, at least one C₁₋₂₀ alkyl acrylate and/or at least one N-mono- or N, N-di (C₂₋₁₂ alkyl) (meth) acrylamide, and acrylic acid and/or methacrylic acid,
(b) at least one thickening agent chosen from the homopolymers and copolymers based on (meth)acrylic acid, which are crosslinked or non-crosslinked, and
(c) at least one co-thickening agent different from (b) chosen from the thickening polymers below:
- non-cellulosic polymers of natural origin,
- synthetic co-thickening agents chosen from polyethylene glycols and their derivatives, as well as the homopolymers and copolymers, crosslinked or not, based on acrylamide or methacrylamide.

2. Cosmetic composition according to Claim 1, **characterized in that** the fixing film-forming polymer is a branched block copolymer comprising, as monomers, n-butyl acrylate, acrylic acid, methacrylic acid and allyl methacrylate.

3. Cosmetic composition according to Claim 1 or 2, **characterized in that** the concentration of fixing film-forming polymer is between 0.1 and 10% by weight, preferably between 1 and 5% by weight, relative to the total weight of the final composition.

4. Cosmetic composition according to any one of the preceding claims, **characterized in that** the thickening agent (b) is a crosslinked acrylic acid homopolymer.

5. Cosmetic composition according to one of Claims 1 to 3, **characterized in that** the thickening agent (b) is a copolymer of acrylic acid or of methacrylic acid and of a C₁₀₋₃₀ alkyl acrylate or methacrylate.

6. Cosmetic composition according to any one of the preceding claims, **characterized in that** the concentration of thickening agent (b) is between 0.1 and 3% by weight, and preferably between 0.2 and 2% by weight, relative to the total weight of the final composition.

7. Cosmetic composition according to any one of the preceding claims, **characterized in that** the co-thickening agent (c) is of natural origin and is chosen from guar, xanthan, scleroglucan, gelan, rhamsan and karaya gums, alginates, maltodextrin, starch and its derivatives, and carob flour.

8. Cosmetic composition according to Claim 7, **characterized in that** the co-thickening agent (c) is chosen from guar gum or xanthan gum.

9. Cosmetic composition according to any one of the preceding claims, **characterized in that** the concentration of the co-thickening agent is between 0.05 and 2% by weight, preferably between 0.1 and 1% by weight, relative to the total weight of the final composition.

10. Use of the cosmetic compositions according to any one of the preceding claims for styling and fixing the hair.

## Patentansprüche

1. Kosmetische Zusammensetzung, enthaltend in einem wäßrigen oder wäßrig-alkoholischen kosmetisch unbedenklichen Träger:
(a) mindestens ein festigendes filmbildendes Polymer, das unter verzweigten Blockcopolymeren, die eine Struktur aus hydrophoben Blöcken, an denen über bifunktionelle Einheiten eine bestimmte Zahl von hydrophilen Blöcken fixiert ist, aufweisen und als Hauptmonomere mindestens ein C₁₋₂₀-Alkylacrylat und/oder mindestens ein N-Mono- oder N,N-Di(C₂-₁₂)alkyl(meth)acrylamid und Acrylsäure und/oder Methacrylsäure enthalten, ausgewählt ist,
(b) mindestens einen Verdicker, der unter gegebenenfalls vernetzten Homopolymeren und Copolymeren auf Basis von (Meth)acrylsäure ausgewählt ist, und
(c) mindestens einen von (b) verschiedenen Coverdicker, der unter den nachstehenden Verdickungspolymeren ausgewählt ist:
- nichtcellulosische Polymere natürlichen Ursprungs;
- synthetische Coverdicker, die unter Polyethylenglykolen und Derivaten davon sowie gegebenenfalls vernetzten Homopolymeren und Copolymeren auf Basis von Acrylamid oder Methacrylamid ausgewählt sind.

2. Kosmetische Zusammensetzung nach Anspruch 1, **dadurch gekennzeichnet, daß** es sich bei dem festigenden filmbildenden Polymer um ein verzweigtes Blockcopolymer handelt, das als Monomere n-Butylacrylat, Acrylsäure, Methacrylsäure und Allylmethacrylat enthält.

3. Kosmetische Zusammensetzung nach Anspruch 1 oder 2, **dadurch gekennzeichnet, daß** die Konzentration des festigenden filmbildenden Polymers zwischen 0,1 und 10 Gew.-% und vorzugsweise zwischen 1 und 5 Gew.-%, bezogen auf das Gesamtgewicht der fertigen Zusammensetzung, liegt.

4. Kosmetische Zusammensetzung nach einem der vorhergehenden Ansprüche, **dadurch gekennzeichnet, daß** es sich bei dem Verdicker (b) um ein vernetztes Homopolymer von Acrylsäure handelt.

5. Zusammensetzung nach einem der Ansprüche 1 bis 3, **dadurch gekennzeichnet, daß** es sich bei dem Verdicker (b) um ein Copolymer von Acrylsäure oder Methacrylsäure und einem C₁₀₋₃₀-Alkylacrylat oder -methacrylat handelt.

6. Kosmetische Zusammensetzung nach einem der vorhergehenden Ansprüche, **dadurch gekennzeichnet, daß** die Konzentration des Verdickers (b) zwischen 0,1 und 3 Gew.-% und vorzugsweise zwischen 0,2 und 2 Gew.-%, bezogen auf das Gesamtgewicht der fertigen Zusammensetzung, liegt.

7. Kosmetische Zusammensetzung nach einem der vorhergehenden Ansprüche, **dadurch gekennzeichnet, daß** der Coverdicker (c) natürlichen Ursprungs ist und unter Guar-Gummi, Xanthan, Skleroglucan-Gummi, Gellan-Gummi, Rhamsan-Gummi, Karaya-Gummi, Alginaten, Maltodextrin, Stärke und Derivaten davon und Johannisbrotkernmehl ausgewählt ist.

8. Kosmetische Zusammensetzung nach Anspruch 7, **dadurch gekennzeichnet, daß** der Coverdicker (c) unter Guar-Gummi und Xanthan ausgewählt ist.

9. Kosmetische Zusammensetzung nach einem der vorhergehenden Ansprüche, **dadurch gekennzeichnet, daß** die Konzentration des Coverdickers (c) zwischen 0,05 und 2 Gew.-% und vorzugsweise zwischen 0,1 und 1 Gew.-%, bezogen auf das Gesamtgewicht der fertigen Zusammensetzung, liegt.

10. Verwendung der kosmetischen Zusammensetzungen nach einem der vorhergehenden Ansprüche zum Frisieren und Festigen der Haare.
